# EUROPEAN PATENT APPLICATION

(11) **EP 1 637 177 A1**
(43) Date of publication of application: **22.03.2006**
(21) Application number: 04745277.6
(22) Date of filing: 21.05.2004
(51) Int. Cl.: A61M 29/02

(54) **TEMPORARILY INDWELLED STENT AND STENT GRAFT**

(30) Priority: 23.05.2003 JP 2003146404
(71) Applicant: Ishimaru, Shin, Tokyo 150-0001 (JP); Kitamura, Yoshiro, Kanazawa-shi Ishikawa 921-8042 (JP)
(72) Inventor: ISHIMARU, Shin, Shibuya-ku, Tokyo 150-0001 (JP); KITAMURA, Atsushi, . (JP)
(74) Representative: Viering, Jentschura & Partner
(86) International application number: PCT/JP2004/006966
(87) International publication number: WO 2004/103451

(57) **Abstract**

There is provided A temporary stent comprising a first contractible and expandable stent body having a loosely-interlocked knitted structure formed by twisting at least one filament W in a spiral fashion and being configured to be expanded to form a cylindrical shape as a normal state; a first contractible and expandable proximal end configured to be expanded to form a tapered shape as a normal state; a middle strut maintained in a contracted configuration; a second contractible and expandable proximal end configured to be expanded to form a tapered shape as a normal state; a second contractible and expandable stent body configured to be expanded to form a cylindrical shape as a normal state; a third contractible and expandable proximal end configured to be expanded to form a tapered shape as a normal state; and a strut, in serial order. The filament W is formed of, for example, a shape-memory material.

## Description

### [TECHNICAL FIELD]

This invention relates to temporarily locating type stents and stent-grafts for use in inspection of stenotic artery diseases, expansive arterial diseases such as an aneurysm or any other diseases, and in emergency situation, and more particularly to temporarily locating type stents and stent-grafts for use in 1) checking if the placement of a permanent stent involves any risk of occurring hindrance of blood flow, 2) ensuring an adequate inner diameter in the area of stenosis within a tubular organ such as an artery, 3) temporarily closing off rhagades occurred in the inner wall of a tubular organ such as an artery, and 4) temporary hemostasis within a blood vessel in case of emergency.

### [BACKGROUND ART]

An aneurysm is an unfavorable condition resulting from arteriosclerosis, inflammation and other causes that will grow larger and lead to fatal ruptures when left unattended. Now that medicinal treatments produce hardly any good results, surgical operations using an artificial brood vessel, for example, excision and replacement of bosselation have conventionally been resorted to. However, this condition appears frequently in aged persons and is often associated with disturbances of a variety of organs (such as the brain, heart and liver). Therefore, applicability of surgical operations, which can often be exorbitant stresses, is limited. Thus, intravascular treatments using catheters whose operative stresses are relatively moderate have been receiving attention of the world. The use of stents is one of such treatments in popular use.

A stent is a term to generally denote the artificial tubular structures that are inserted in blood vessels and other tubular organs in the body to support them. When, for example, stenosis or other defamation has occurred in blood vessels, gallbladers, gullets, bowels, ureters and other internal tubular organs, a stent is inserted in them to prevent the recurrence of such stenosis or deformation by supporting them. The stent which has been widely applied clinically in order to treat stenotic artery diseases has a cylindrical shape formed of a wire mesh or spring made of stainless steel or shape memory alloy (NiTi). The stent is designed so that it is repeatedly contractible and expandable in a radial direction. The above mentioned stent is inserted into an artificial blood vessel (i.e., a graft) made of polyester or fluoropolymer and is fastened by stitching it to the artificial blood vessel to form a blood vessel inserted-type artificial blood vessel. This blood vessel inserted-type artificial blood vessel is also referred to as a stent-graft, which has been effectively used to treat the aneurysm. These stents and stent-grafts are well known worldwide. See References 1 through 6.

These stents or stent-grafts can be delivered to the predetermined location inside a blood vessel by a catheter. Specifically, the stent or stent-graft is compressed to have a diameter smaller than the inner diameter of a fine catheter and is contained in the catheter. The stent or stent-graft is inserted from the incised part of a peripheral artery (mainly the femoral artery) into the location of lesion within the blood vessel. Subsequently, the stent or stent-graft is pushed out from the catheter to radically expand to the desired diameter. The stent or stent-graft is further positioned in the location of lesion within the blood vessel, thereby allowing enlargement of the area of stenosis, occlusion of the aneurysm and repair of blood flow. The intravascular treatment using stents or stent-grafts causes only mild operative stresses. Therefore, it can lighten the burden imposed on a patient, and can be also applied to the aneurysm to which it has not been applied. Thus, this treatment is an effective for patients suffering from the stenotic artery diseases and other expansive arterial diseases.

However, conventional stents or stent-grafts proposed so far cannot be retracted and recovered after they have been once released from catheters and allowed to expand on their own. As such, they present a serious problem that they cannot be recovered from within the blood vessel even after any organic or blood flow disorder has occurred. In addition, if they have been placed in the wrong place, they are difficult to move to the right place for correction.

To securely close off an aneurysm by the use of a conventional stent or stent-graft, it is necessary to keep the conventional stent or stent-graft tightly fitted in the artery by ensuring that the stent or stent-graft covers not only the aneurysm itself but also the normal arteries at its center and periphery. However, this can sometimes block up main arteries branching off from the vicinity of the aneurysm, which can lead to other organic disorder. For example, the renal, inferior mesenteric and hypogastric arteries are the main visceral arteries affected by the abdominal aortic aneurysm. Occlusion of these arteries can lead to renal and intestinal ischemic dysfunctions. With a thoracic aortic aneurysm, occlusion of the intercostals artery can cause disturbance of the spinal cord blood flow leading to a serious complication called paraplegia (lower-body motor paralysis).

To solve these drawbacks, the inventors of this invention have proposed that a stent or stent-graft which can recovered from the body is temporarily positioned in the location of lesion within a blood vessel before the above mentioned conventional stent or stent-graft is permanently positioned therein. In this case, if the location in which hindrance of blood flow may occur when the conventional stent or stent-graft is inserted within the blood vessel can be confirmed in advance, the conventional stent can be positioned in the safe location of lesion within the blood vessel. On the basis of this idea, the inventors of this invention have proposed a temporarily locating type stent or stent-graft (hereinafter, referred to as a "temporary stent or stent-graft") which can be recovered from a body after it has been released from a catheter and positioned in the location of lesion within a blood vessel. See reference 7.

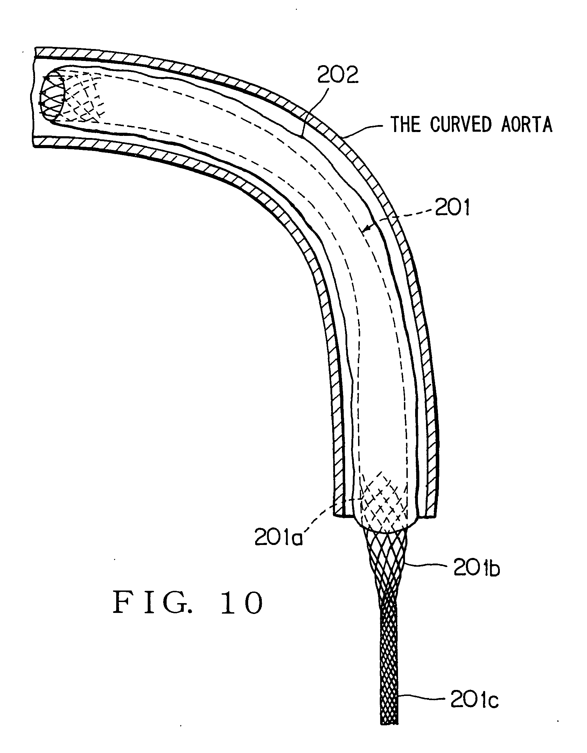

FIG. 8 is an illustration of a conventional temporary stent-graft which has been pushed out from a catheter and maintains its expanded condition. Referring to FIG. 8, a reference numeral 111 denotes a temporary stent proposed by the inventor of this invention. The temporary stent 111 is composed of a stent body 112 in the forward portion and a mast wire 118 in the rear portion. The stent body 112 has three elastic rings 114 each formed in ring shape by a metallic wire bent in zigzag. These elastic rings 114 each are made of stainless steel, titanium, shape memory alloy, etc. Around the elastic ring 114, eight coupling wires 115 are arranged at regular intervals in a circumferential direction. These coupling wires 115 are welded or soldered at a point of cross-section of the elastic rings 114 to form an elastic cylindrical body 113, which is also covered with a graft 129 made of filament material including polyester, polyurethane, fluoropolymer, etc. The graft 129 and the three elastic rings 114 are sutured together and fixed to each other.

The elastic cylindrical body 113 is consecutively arranged with a plurality of parallel portions 116 via coupling wires 115. The mast lines 118 whose tips 119 are connected to the parallel portions 116 in the stent body 112 extend rearward in a bundle. The coupling wires 115 and mast wires 118 are made of shape memory alloy. The bundle of the mast wires 118 has a diameter which is slightly smaller than the inner diameter of a catheter 131. The mast wires 118 have a length enough to penetrate through the catheter 131 and to allow easy manipulation from outside of a patient when the stent body 112 is positioned at a predetermined location within a blood vessel. The stent body 112 and the mast wires 118 have rigidity enough to be pushed out from the catheter 131 by the manipulation from outside of the body and to be retracted back into the catheter 131. The catheter 131 is provided with a bent portion at its tip 132 such that the stent body 112 can be smoothly contracted and be contained therein.

Once contracted radially, the elastic cylindrical body 113 covered with the graft 129 is smaller in diameter than the inner diameter of the catheter 131 in which it is to be contained. The stent body 112 is designed to have an expanded outer diameter of about 20 mm to 40 mm such that it can sufficiently enlarge the inner wall of the blood vessel after pushed out from the catheter 131.

The conventional temporary stent, when implanted into an internal tubular organ which is relatively less curved, would not cause a serious problem. Meanwhile, the stent which has been inserted through a sharply curved internal tubular organ in order to treat stenotic artery diseases, expansive arterial diseases (for example, an aneurysm) or other diseases suffers from a number of disadvantages as follow.

The conventional temporary stent or stent-graft, during insertion through the sharply curved internal tubular organ (for example, the aortic arch, the distal aortic arch, the distal descending aorta and the abdominal aorta) can be traumatic to the inner wall of the said organ. This is because the bent portions of the elastic rings which are adjacent in the stent are liable to contact or crossover with respect to each other within the curved inner wall of the said organ. As such, the graft portion of the conventional temporary stent or stent-graft can also wrinkle leading to hindrance of blood flow, and if things come to the worst, thrombus formation. Furthermore, when the conventional temporary stent or stent-graft is positioned within the sharply curved internal tubular organ, it bends along the curve of the internal tubular organ and is tightly fixed to the inner wall of the internal tubular organ. Accordingly, when the conventional temporary stent or stent-graft, after completion of inspection or treatment, is intended to be retracted back into a catheter, the graft portion irritates the inner wall, and thus presents a high risk of unnecessary trauma to the inner wall.

To solve the above-discussed problems, the inventors of this invention have proposed an improved temporary stent-graft. See reference 8. Fig. 9 is a view for illustrating another conventional temporary stent-graft which has been proposed by the inventors of this invention. As shown in Fig. 9, a conventional temporary stent-graft 210 comprises a stent 201 having a loosely-interlocked knitted structure formed by twisting at least one filament in a spiral fashion and a graft 202. Concerning the temporary stent-graft 210, 1) the stent 201 comprises a stent body 210a which is freely expandable or contractible and is expanded to form a cylindrical shape as a normal state, a proximal end 201b which is consecutively arranged with the stent body 201a and is expanded to form a tapered shape as a normal state, and a strut 201 which is consecutively arranged with the proximal end 201b and is maintained in a contracted configuration; 2) the stent body 201a is covered with the graft 202; and 3) a distal end 202a of the graft 202 is coupled with a distal end of the stent 201 by a connecting member 203 in a manner that can be reversed.

Fig. 10 is a view for illustrating the above discussed conventional temporary stent-graft which has been inserted through the curved aorta. In such a case, as shown in Fig. 10, the middle area of the stent body 201 a formed of the loosely-interlocked knitted structure bends to result in a narrowing of its diameter. Accordingly, a distal end 201d and the proximal end 201b are continuously deformed so that the stent-graft can not be sufficiently expanded. In such a case, blood flow, when flowed into between the curved aorta and the stent-graft 210 from center or periphery, prevents the stent-graft 210 from tight fixation to the inner wall of the aorta, and therefore, the desired object that the stent-graft is made to block blood flow by its expansion cannot be attained.

Moreover, even if such a conventional loosely-interlocked knitted stent (not shown) is implanted into the curved internal tubular organ (for example, the aorta), the distal end and the proximal end of the stent are continuously deformed so that the stent cannot be sufficiently expanded. Thus, as indicated by the afore-mentioned conventional temporary stent, the stent also cannot enlarge the area of stenosis sufficiently.

Reference 1: Published patent application H7-24072

Reference 2: Published patent application H7-47134

Reference 3: Japanese translation of PCT international application H7-500272

Reference 4: Japanese translation of PCT international application H8-299456

Reference 5: Japanese translation of PCT international application H8-502428

Reference 6: Japanese translation of PCT international application H8-511487

Reference 7: Published patent application H10-337333

Reference 8: Published patent application 2001-333987

### [DISCLOSURE OF THE INVENTION]

To solve above mentioned problems, the present invention is provided.

It is the purpose of the present invention to provide a temporary stent (also referred to as a "temporarily locating type stent") which can be sufficiently expanded during insertion through a sharply curved internal tubular organ, can be tightly secured to the inner wall of the internal tubular organ, and can be expanded or contracted with no trauma to the inner wall.

According to the invention as set forth in claim 1 of the appended claims, there is provided a temporary stent comprising, a first contractible and expandable stent body having a loosely-interlocked knitted structure formed by twisting at least one filament in a spiral fashion and being configured to be expanded to form a cylindrical shape as a normal state; a first contractible and expandable proximal end configured to be expanded to form a tapered shape as a normal state; a middle strut maintained in a contracted configuration; a second contractible and expandable proximal end configured to be expanded to form a tapered shape as a normal state; a second contractible and expandable stent body configured to be expanded to form a cylindrical shape as a normal state; a third contractible and expandable proximal end configured to be expanded to form a tapered shape as a normal state; and a strut in serial order.

According to the invention as set forth in claim 2 of the appended claims, there is provided the temporary stent of claim 1 characterized in that the strut has a loosely-interlocked knitted structure formed by twisting at least one filament in a spiral fashion, and is maintained in its contracted configuration.

According to the invention as set forth in claim 3 of the appended claims, there is provided the temporary stent of claim 1 characterized in that the strut is a rod-shaped body formed of a resin material including polytetrafluoroethylene, polyolefin, polyester, polyurethane, polysiloxane, etc.

According to the invention as set forth in claim 4 of the appended claims, there is provided the temporary stent of any one of claims 1-3 characterized in that the filament is formed of a shape-memory material such that the first and second stent bodies each can be expanded to form cylindrical shapes as their normal state, the first, second and third proximal ends each can be tapered as their normal state, and the middle strut can be maintained in its contracted configuration as a normal state.

According to the invention as set forth in claim 5 of the appended claims, there is provided the temporary stent of claim 1 or 2 characterized in that the filament is formed of a shape-memory material such that the first and second stent bodies each can be expanded to form cylindrical shapes as their normal state, the first, second and third proximal ends each can be tapered as their normal state, and the middle strut and the strut each can be maintained in their contracted configurations as their normal state.

According to the invention as set forth in claim 6 of the appended claims, there is provided the temporary stent of claim 4 or 5 characterized in that the shape-memory material includes Ni-Ti based shape-memory alloy, Cu-Al-Ni based shape-memory alloy, or Cu-Zn-Al based shape-memory alloy.

According to the invention as set forth in claim 7 of the appended claims, there is provided the temporary stent of any one of claims 1-3 characterized in that the filament is formed of a metal wire including stainless steel, titanium, nickel or tantalum.

According to the invention as set forth in claim 8 of the appended claims, there is provided the temporary stent of any one of claims 1-3 characterized in that the filament is formed of a plastic wire or fiber reinforced plastic wire including polytetrafluoroethylene, polyolefin, polyester, polyurethane or polysiloxane.

According to the invention as set forth in claim 9 of the appended claims, there is provided the temporary stent of any one of claims 1-8 characterized in that the first stent body has a length of 5 cm to 10 cm, the first proximal end has a length of 2 cm to 3 cm, the middle strut has a length of 4 cm to 6 cm, the second proximal end has a length of 2 cm to 3 cm, the second stent body has a length of 5 cm to 10 cm, the third proximal end has a length of 4 cm to 6 cm, and the strut has a length of 60 cm to 100 cm.

According to the invention as set forth in claim 10 of the appended claims, there is provided the temporary stent of any one of claims 1-9 characterized by being contained in a pipe-shaped catheter.

According to the invention as set forth in claim 11 of the appended claims, there is provided a temporary stent-graft comprising the stent of any one of claims 1-10 and a cylindrically shaped graft configured to cover the stent in the area ranging between the first stent body and the second stent body.

According to the invention as set forth in claim 12 of the appended claims, there is provided the temporary stent-graft of claim 11 characterized in that the distal end of the graft is coupled with the distal end of the stent by a connecting member in a manner that can be reversed.

According to the invention as set forth in claim 13 of the appended claims, there is provided the temporary stent-graft of claim 12 characterized in that the distal end of the stent is provided with a loop formed by turning around a plurality of filaments arranged at regular intervals, putting two filaments together end on end, and twisting the turn-around part of the filament.

According to the invention as set forth in claim 14 of the appended claims, there is provided the temporary stent-graft of claim 12 or 13 characterized in that the connecting member is a suture.

According to the invention as set forth in claim 15 of the appended claims, there is provided the temporary stent-graft of any one of claims 11-14 characterized by being contained in a pipe-shaped catheter.

### [INDUSTRIAL APPLICABILITY]

According to the invention as set forth in any one of claims 1-3, 7-9 of the appended claims, there is provided "a middle strut maintained in a contracted configuration" which is located between the first and second stent bodies each having conventional loosely-interlocked knitted structures, and has a smaller diameter than the first and second stent bodies. Accordingly, even if the stent is inserted into a sharply curved internal tubular organ, for example, the aorta, its middle strut configured to maintain a contracted state can readily adapt to the curve in the internal tubular organ. In addition, since both the first and second stent bodies can hold their great dilating forces, the stent is allowed to be tightly secured to the inner wall of the internal tubular organ, resulting in enlargement of the area of stenosis with no trauma to the inner wall.

According to the invention as set forth in any one of claims 4-6 of the appended claims, the filament is formed of a shape memory material such that the first and second stent bodies each can be expanded to form cylindrical shapes as their normal state, the first, second and third proximal ends each can be tapered as a normal state, and the middle strut and the strut each can be maintained in their contracted configurations as their normal state.

According to the invention as set forth in claim 10 of the appended claims, the stent is contained in the pipe-shaped catheter, and therefore it is suitable for use in emergency situation.

According to the invention as set forth in claim 11 of the appended claims, the cylindrically shaped graft is configured to cover the stent of any one of claims 1-10 in the area ranging between the first stent body and the second stent body in the temporary stent-graft. That is to say, this stent-graft is characterized by "a middle strut maintained in a contracted configuration" which is located between the first and second stent bodies each having conventional loosely-interlocked knitted structures, and has a smaller diameter than the first and second stent bodies. Owing to these characteristics, even if the stent-graft is to be positioned within the sharply curved internal tubular organ (for example, the aortic arch, the distal aortic arch, the distal descending aorta and the abdominal aorta), it can adapt to the curve in said organ through repeated release and retraction. Furthermore, in case the stent-graft is temporarily positioned in said organ, the middle strut maintained in a contracted configuration can cope with the curve within the internal tubular organ and both the first and second stent bodies can hold their great dilating forces, resulting in prevention of inflow of body fluid (for example, blood) from either center or periphery. Accordingly, the stent-graft can be tightly secured to the inner wall of the internal tubular organ with no trauma to the inner wall.

With respect to an aneurysm occurred within a curved artery and presenting a high risk of developing organ ischemia, the temporary stent-graft as set forth in claim 11 of the appended claims enables one to inspect the risk associated with development of organ ischemia in advance, which has never been done before by conventional technologies. Accordingly, the stent-graft in accordance with the present invention substantially broadens the field which permanent stents or stent-grafts can be applied to, and thus can provide a variety of therapeutic advantages for a number of patients suffering from the aneurysm, etc.

According to the invention as set forth in claim 12 of the appended claims, the distal end of the graft is coupled with the distal end of the stent by the connecting member in a manner that can be reversed, and therefore, after checking if spinal cord ischemia, abdominal main organ ischemia, and so on occur by locating the stent-graft in the aneurysm for a given time, the first and second stent bodies and the first, second and the third proximal ends of the stent are retracted back into the catheter. In this state, the graft which has been tightly secured to the inner wall within the blood vessel can be gradually reversed from at its distal end (i.e., a portion fixed by the connecting member) and then can be removed with no trauma to the inner wall.

According to the invention as set forth in claim 13 of the appended claims, since the distal end of the stent-graft is provided with a loop formed by turning around a plurality of filaments arranged at regular intervals, putting two filaments together end on end, and twisting the turn-around part of the filament, it can be coupled with the distal end of the stent by the connecting member in a manner that can be reversed. Moreover, since such a distal end formed of a plurality of loops is smooth, the stent-graft is not traumatic to the inner wall of the blood vessel.

According to the invention as set forth in claim 14 of the appended claims, since the connecting member is a suture, the stent-graft is not traumatic to the inner wall of the blood vessel.

According to the invention as set forth in claim 15 of the appended claims, since the stent-graft of any one of claims 11-14 is contained in the pipe-shaped catheter, the stent-graft which has been previously subjected to sterilization is suitable for use in emergency situation.

### [BEST MODE FOR CARRYING OUT THE INVENTION]

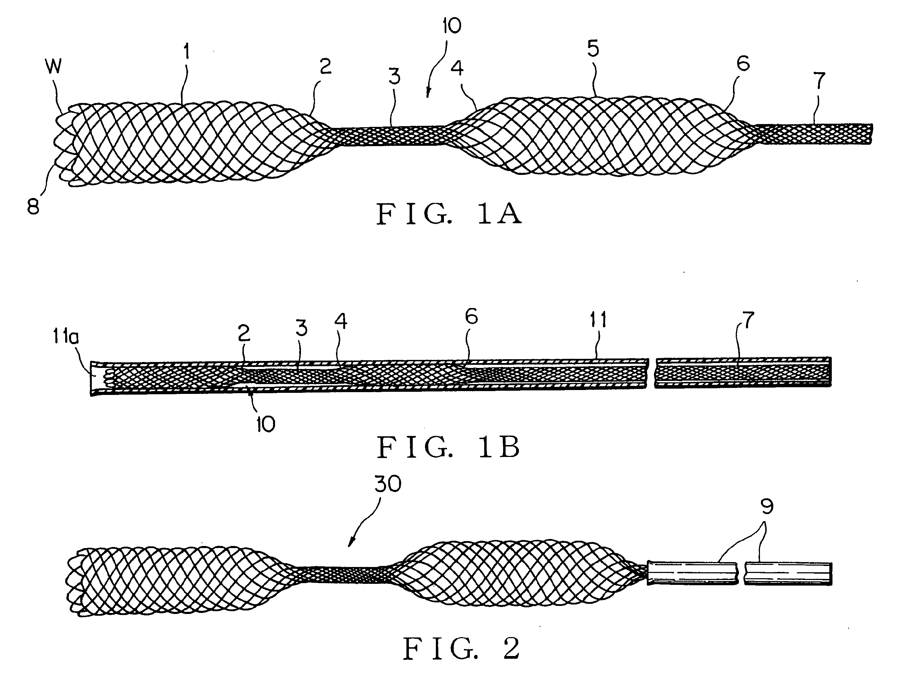

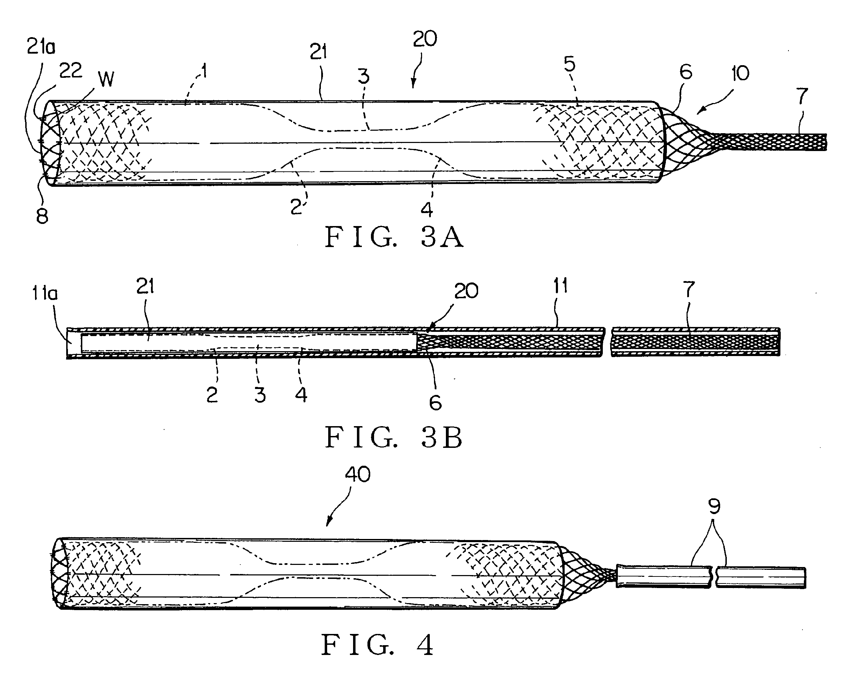

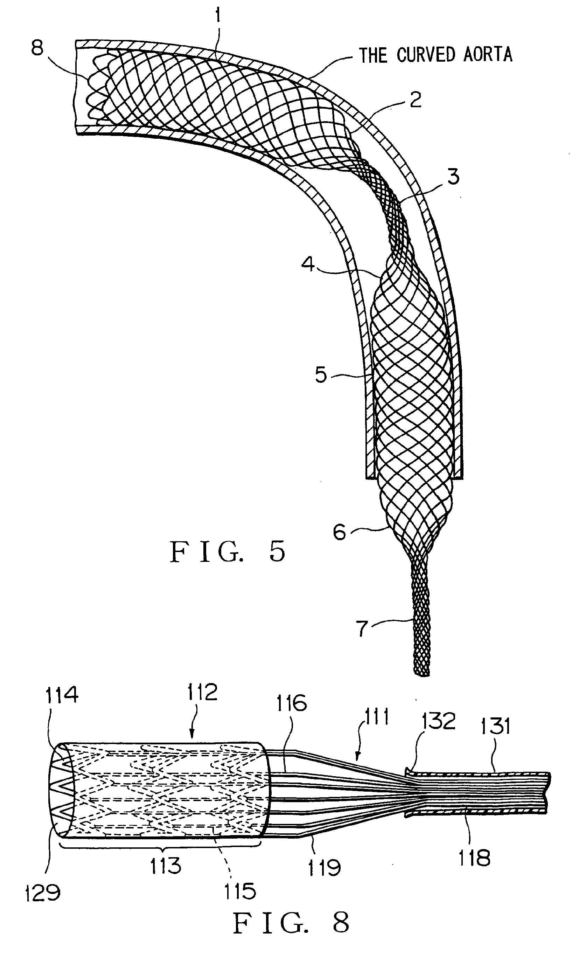

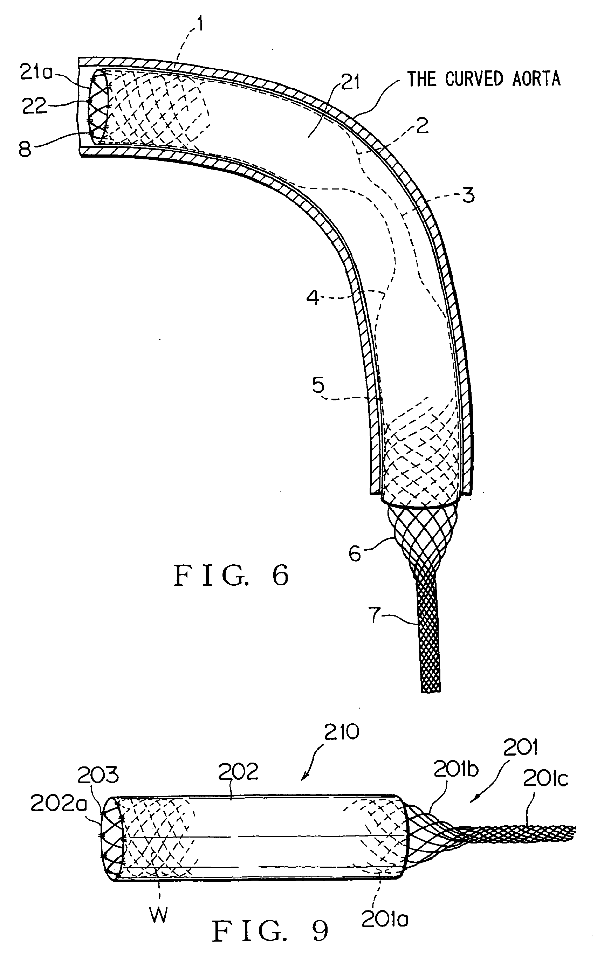

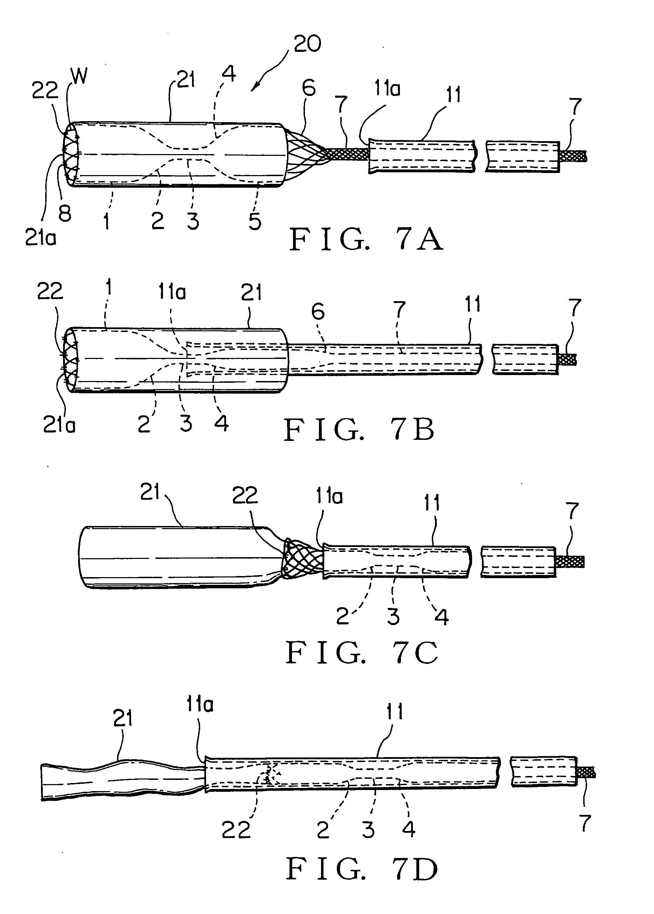

The present invention will now be described by way of example with reference to the accompanying figures. FIGS. 1A and 1B are illustrations of one embodiment of the temporary stent in accordance with the present invention. FIG. 1A is an illustration of the temporary stent, and FIG. 1B is an illustration of the temporary stent which has been contained in a catheter. FIG. 2 is an illustration of another embodiment of the temporary stent in accordance with the present invention. FIGS. 3A and 3B are illustrations of one embodiment of the temporary stent-graft in accordance with the present invention. FIG. 3A is an illustration of the temporary stent-graft, and FIG. 3B is an illustration of the temporary stent-graft which has been contained in a catheter. FIG. 4 is an illustration of another embodiment of the temporary stent-graft in accordance with the present invention. FIG. 5 is an illustration of the temporary stent-graft as depicted in FIGS. 1A and 1B which has been inserted into the curved aorta. FIG. 6 is an illustration of the temporary stent as depicted in FIGS. 3A and 3B which has been inserted into the curved aorta. FIGS. 7A through 7D are illustrations of yet another embodiment of the temporary stent-graft in accordance with the present invention. FIG. 7A is an illustration of the manner in which the stent-graft is pushed out from a catheter. FIG. 7B is an illustration of the manner in which the stent-graft is about to be contained in the catheter. FIG. 7C is an illustration of the manner in which the stent-graft is almost contained in the catheter while its graft portion is in a reversed state. FIG. 7D is an illustration of the manner in which the reversed graft portion is about to be contained in the catheter.

Referring now to FIG. 1A, a reference numeral 10 designates a temporary stent. The temporary stent 10 comprises a first contractible and expandable stent body 1 having a loosely-interlocked knitted structure formed by twisting at least one filament W in a spiral fashion, and being configured to be expanded to form a cylindrical shape as a normal state; a first contractible and expandable proximal end 2 configured to be expanded to form a tapered shape as a normal state; a middle strut 3 maintained in a contracted configuration; a second contractible and expandable proximal end 4 configured to be expanded to form a tapered shape as a normal state; a second contractible and expandable stent body 5 configured to be expanded to form a cylindrical shape as a normal state; a third contractible and expandable proximal end 6 configured to be expanded to form a tapered shape as a normal state; and a strut 7 in series. Preferably, the strut 7 is made of a loosely-interlocked knitted structure formed by twisting at least one filament in a spiral fashion, and is maintained in its contracted configuration. Referring to FIG. 2, a strut 9 may be a rod-shaped body formed of a resin material including polytetrafluoroethylene, polyolefin, polyester, polyurethane, polysiloxane, etc. Preferably, the rod-shaped body is pipe-shaped. The edge of the proximal end 6 is, for example, pushed into the tip of the rod-like body, and then is fixed thereto.

Referring to FIG. 5, since a temporary stent 10 is provided with "a middle strut maintained in a contracted configuration" which is located between the first and second stent bodies each having conventional loosely-interlocked knitted structures, and has a smaller diameter than the first and second stent bodies (see Reference 8), the middle strut maintained in a contracted configuration can readily adapt to the curve in the internal tubular organ (for example, the aorta) where the stent is implanted, and at the same time both the first and second stent bodies 1, 5 can continue to provide great dilating forces in the implanted state. As a result, the temporary stent 10 is allowed to be tightly secured to the inner wall of the internal tubular organ, resulting in enlargement of the area of stenosis with no trauma to the inner wall. Referring to FIG. 5, reference numerals 2, 4, 6 each designate the proximal ends configured to be expanded to form tapered shapes as their normal state respectively, and a reference numeral 7 designates a strut.

The temporary stent 10 in accordance with the present invention preferably comprises the filament W formed of a shape-memory material such that the first and second stent bodies 2, 5 each can be expanded to form cylindrical shapes as their normal state, the first, second and third proximal ends 2, 4, 6 each can be tapered as their normal state, and the middle strut 3 can be maintained in its contracted configurations at its normal state. More preferably, with respect to the stent 10, the filament W is formed of a shape-memory material such that the first and second stent bodies 2, 5 each can be expanded to form cylindrical shapes as their normal state, the first, second and third proximal ends 2, 4, 6 each can be tapered as their normal state, and the middle strut 3 and the strut 7 each can be maintained in their contracted configuration at their normal state. In case the filament W is formed of a shape-memory material such that the first and second stent bodies 2, 5 each can be expanded to form cylindrical shapes as their normal state, the first, second and third proximal ends 2, 4, 6 each can be tapered as their normal state, and the middle strut 3 and the strut 7 each can be maintained in their contracted configurations at their normal state, it is possible to prepare the stent 10 having such a complicated structure with ease as well as with low cost.

The shape-memory material suitable for use in the present invention preferably includes Ni-Ti based shape-memory alloy, Cu-Al-Ni based shape-memory alloy, or Cu-Zn-Al based shape-memory alloy.

In the temporary stents 10, 30, the filament W may be formed of a metal wire including stainless steel, titanium, nickel or tantalum, or plastic wire or fiber reinforced plastic wire including synthetic resin material such as polytetrafluoroethylene, polyolefin, polyester, polyurethane or polysiloxane. Furthermore, the filament W made of the afore-mentioned shape-memory alloy or any other suitable metal material may be coated with the afore-mentioned synthetic resin material.

With respect to the temporary stents 10, 30, the first stent body 1 has a length of 5 cm to 10 cm, the first proximal end 2 has a length of 2 cm to 3 cm, the middle strut 3 has a length of 4 cm to 6 cm, the second proximal end 4 has a length of 2 cm to 3 cm, the second stent body 5 has a length of 5 cm to 10 cm, the third proximal end 6 has a length of 4 cm to 6 cm, and each of the struts 7, 9 has a length of 60 cm to 100 cm.

As shown in FIG. 1B, the temporary stents 10, 30 may be previously contained in a pipe-shaped catheter 11. The stents 10, 30 which have been subjected to sterilization can be advantageously used in emergency situation. The temporary stents 10, 30 may be contained immediately before use.

Refrerring to FIG. 3A, a reference numeral 20 designates a temporary stent-graft. The stent-graft 20 comprises the stent 10 having a loosely-interlocked knitted structure formed by twisting at least one filament W in a spiral fashion, and a cylindrical graft 21 (i.e., an artificial blood vessel) covering the stent 10. The stent 10 is provided with the first contractible and expandable stent body 1 configured to be expanded to form a cylindrical shape as a normal state; the first contractible and expandable proximal end 2 configured to be expanded to form a tapered shape as a normal state; the middle strut 3 maintained in a contracted configuration; the second contractible and expandable proximal end 4 configured to be expanded to form a tapered shape as a normal state; the second contractible and expandable stent body 5 configured to be expanded to form a cylindrical shape as a normal state; and the third contractible and expandable proximal end 6 configured to be expanded to form a tapered shape as a normal state; and a strut 7 in series. Preferably, the strut 7 has a loosely-interlocked knitted structure formed by twisting at least one filament in a spiral fashion, and is maintained in a contracted configuration. As shown in FIG. 4, the strut 9 may be a rod-shaped body formed of a resin material such as polytetrafluoroethylene, polyolefin, polyester, polyurethane, polysiloxane, etc. Preferably, the rod-shaped body is pipe-shaped. The edge of the proximal end 6 is, for example, pushed into the tip of the rod-shaped body, and then is fixed thereto. Referring to FIG. 4, a reference numeral 40 designates a temporary stent-graft. Each of the stents 10, 30 is covered with a cylindrical graft 21 in the area ranging between the first stent body 1 and the second stent body 5.

Concerning the temporary stent-grafts 20, 40, the temporary stent as set forth in any one of claims 1-7 is covered with the cylindrical graft in the area ranging between the first stent body 1 and the second stent body 5. Since the stent 10 constituting the stent-graft 20 is provided with "a middle strut 3 maintained in a contracted configuration" which is located between the first and second stent bodies each having conventional loosely-interlocked knitted structures, and has a smaller diameter than the first and second stent bodies (see Reference 8), as shown in FIG. 6, the middle strut 3 configured to maintain its contracted configuration can readily adapt to a curve in an internal tubular organ (for example, the aortic arch, the distal aortic arch, the distal descending aorta and the abdominal aorta) where the stent-graft is implanted, through repeated release and retraction of the stent-graft, and at the same time both the first and second stent bodies 1, 5 can continue to provide great dilating forces in the implanted state. As a result, both the first and second stent bodies 1, 5 which are covered with the graft 21 are expanded so as to prevent inflow of body fluid (for example, blood) from either center or periphery, and therefore the temporary stent-graft can be tightly secured to the inner wall of the internal tubular organ, resulting in enlargement of the area of stenosis with no trauma to the inner wall.

Concerning the temporary stent-grafts 20, 40, in case the stent-graft is to be temporarily located inside a thoracic aortic aneurysm, the occurrence of spinal cord ischemia which results from hemostasis in the intercostals artery branching off from the aorta thoracica is continuously monitored over a given period of time by using a spinal evoked potential measuring device. Moreover, in case the stent-graft is to be temporarily located inside an abdominal aortic aneurysm, any changes in urine volume or the occurrence of intestinal ischemia which results from hemostasis in the artery branching off from the abdominal aorta is also monitored. After confirming there is no abnormality in urine volume or there is no sign of either spinal cord ischemia or intestinal ischemia, a permanent stent or stent-graft (not shown) can be positioned in a safe region inside the aorta with great accuracy. Accordingly, the temporary stent-grafts 20,40 enable one to estimate in advance a possibility that the aneurysm which has occurred in the curved aorta can leads to organ ischemia. As a result, the temporary stent-graft in accordance with the present invention can substantially broaden the fields which permanent stents or stent-grafts can be applied to, and therefore can provide a variety of therapeutic advantages for a number of patients suffering from aneurysm, etc.

Concerning the temporary stent-grafts 20, 40, the distal end 21a of the graft 21 is desired to be coupled with the distal end 8 of the stent 10 by a connecting member 22 in a manner that can be reversed.

Concerning the temporary stent-grafts 20, 40, after checking occurrence of spinal cord ischemia or abdominal ischemia by positioning the temporary stent-graft in the aneurysm over a given period of time, one can push out the catheter 11 to retract the first stent body 1, the first proximal end 2, the middle strut 3, the second proximal end 4, the second stent body 5, and the third proximal end 6 of the stent assembly 10 back into the catheter 11. At this state, the graft 21 which has been tightly secured to the inner wall of the blood vessel can be removed therefrom while being gradually reversed from its distal end 21 a (a portion fixed by the connecting member 22) with no trauma to the inner wall.

The distal end 8 of the stent 10 is formed with a loop formed by turning around a filament (see the turn-around part of the filament W of the distal end 8 in FIG. 1A), putting two filaments together end on end, and twisting the turn-around part of the filament. "A loop formed by turning around a filament" can be prepared by knitting the filament W in a loosely-interlocked fashion so as to produce the turn-around part of the filament W. "A loop formed by putting two filaments together end on end" can be prepared by knitting the filament W to form a loosely-interlocked knitted structure, cutting the loosely-interlocked knitted structure thus obtained transversely, and welding or soldering two adjacent filaments W, W end on end. "A loop formed by twisting the turn-around part of the filament" is prepared by twisting the turn-around part of the filament W. According to the present invention, the loop cannot be limited to afore-mentioned loops, and also includes any loop prepared by other suitable means.

Since the distal end 8 of the stent 10 is provided with a plurality of loops which are arranged at regular intervals, the distal end 21a of the graft 21 can be coupled with the distal end 8 of the stent 10 by the connecting member 22 in a manner that can be reversed. Moreover, since the distal end 8 formed of a plurality of loops is smooth, the stent-graft 20 is not traumatic to the inner wall of the internal tubular organ.

The connecting member 22 is desired to be suture material. In case the connecting member 22 is made of elastic suture material, the suture material is not traumatic to the inner wall of the blood vessel. The connecting member 22 includes, but not limited to paste or heating type adhesive.

The temporary stent-grafts 20, 40, as shown in FIG. 3B, may be previously contained in the pipe-shaped catheter 11. If the stents 20, 40 are previously contained in the catheter 11 in a sterilized state, they can be advantageously used in emergency situation. The stent-grafts 20, 40 may also be contained in the catheter 11 immediately before use.

There is now illustrated operation of the temporary stent-graft in accordance with the present invention.

As shown in FIG. 3A, the graft 21 is configured to cover the temporary stent 10 in the area ranging between the first stent body 1 and the second stent body 5, and is fixed to the distal end 8 of the stent 10 at its distal end 21a by the connecting member 22 in a manner that can be reversed. As shown in FIG. 3B, such a stent-graft is thereafter folded, compressed and contained in the catheter 11.

As shown in FIG. 7A, the temporary stent-graft 20 contained in the catheter 11 is implanted along a guide wire (not shown) which has been previously positioned before incision of peripheral artery, mainly the femoral artery. After arriving at the aneurysm inside the curved aorta, the temporary stent-graft 20 which has been covered with the graft 21 is pushed out from the catheter 11 by pulling the catheter 11 while manually supporting the strut 7 with a length of 60 cm to 100 cm against its arbitrary movement. Subsequently, the stent-graft 20 will self-expand to the relaxed condition and tightly secure the graft 21 to the wall of the blood vessel (see FIG. 6).

As shown in FIG. 7B, after checking occurrence of spinal cord ischemia or abdominal ischemia by temporarily positioning the temporary stent-graft 20 in the aneurysm over a given period of time, the catheter 11 is compressed into the graft 21 until the tip 11a of the catheter 11 reaches the vicinity of the connecting member 22 while the strut 7 being supported. Subsequently, by pulling the strut 7, the stent 10 is retracted back into the catheter 11 in order of the third proximal end 6, the second stent body 5, the second proximal end 4, the middle strut 3, the first proximal end 2, and the first stent body 1.

As shown in FIG. 7C, by pulling both the catheter 11 and the strut 7 at the same time while maintaining the third proximal end 6, the second stent body 5, the second proximal end 4, the middle strut 3, the first proximal end 2, and the first stent body 1 of the stent 10 in the catheter 11, the graft 21 which has been tightly secured to the inner wall of the blood vessel can be detached therefrom while being gradually reversed from its distal end (i.e., the portion fixed by the connecting member 22).

As shown in FIG. 7D, by pulling the strut 7 while maintaining the catheter 11 in its fixed state, the reversed graft 21 is retracted back into the catheter 11 through its tip 11a.

After confirming there is no sign of organ ischemia, permanent stents or stent-grafts can be properly positioned in a safe region within a treated artery.

### [BRIEF DESCRIPTION OF THE DRAWINGS]

FIGS. 1A and 1B are illustrations of one embodiment of the temporary stent in accordance with the present invention. FIG. 1A is an illustration of the temporary stent, and FIG. 1B is an illustration of the temporary stent which has been contained in a catheter.
FIG. 2 is an illustration of another embodiment of the temporary stent in accordance with the present invention.
FIGS. 3A and 3B are illustrations of one embodiment of the temporary stent-graft in accordance with the present invention. FIG. 3A is an illustration of the temporary stent-graft, and FIG. 3B is an illustration of the temporary stent-graft which has been contained in a catheter.
FIG. 4 is an illustration of another embodiment of the temporary stent-graft in accordance with the present invention.
FIG. 5 is an illustration of the temporary stent as depicted in FIGS. 1A and 1B which has been inserted into the curved aorta.
FIG. 6 is an illustration of the temporary stent-graft as depicted in FIGS. 3A and 3B which has been inserted into the curved aorta.
FIGS. 7A through 7D are illustrations of yet another embodiment of the temporary stent-graft in accordance with the present invention. FIG. 7A is an illustration of the manner in which the stent-graft is pushed out from a catheter. FIG. 7B is an illustration of the manner in which the stent-graft is about to be contained in the catheter. FIG. 7C is an illustration of the manner in which the stent-graft is almost contained in the catheter while its graft portion is in a reversed state. FIG. 7D is an illustration of the manner in which the reversed graft portion is about to be contained in the catheter.
FIG. 8 is an illustration of one conventional temporary stent-graft which has been pushed out from a catheter and maintains its expanded condition.
FIG. 9 is an illustration of another conventional temporary stent-graft.
FIG. 10 is an illustration of the conventional temporary stent-graft as depicted in FIG. 9 which has been inserted into the curved aorta.

1: a first stent body
2: a first proximal end
3: a middle strut
4: a second proximal end
5: a second stent body
6: a third proximal end
7, 9: a strut
8: a distal end of stent
10, 30: a stent
11: a catheter
11 a: a tip of catheter
20, 40: a stent-graft
21: a graft
21a: a distal end of graft
22: a connecting member
W: a filament

## Claims

1. A temporary stent, comprising:
a first contractible and expandable stent body having a loosely-interlocked knitted structure formed by twisting at least one filament in a spiral fashion and being configured to be expanded to form a cylindrical shape as a normal state;
a first contractible and expandable proximal end configured to be expanded to form a tapered shape as a normal state;
a middle strut maintained in a contracted configuration;
a second contractible and expandable proximal end configured to be expanded to form a tapered shape as a normal state;
a second contractible and expandable stent body configured to be expanded to form a cylindrical shape as a normal state;
a third contractible and expandable proximal end configured to be expanded to form a tapered shape as a normal state; and
a strut, in serial order.

2. The temporary stent of claim 1, wherein the strut has a loosely-interlocked knitted structure formed by twisting at least one filament in a spiral fashion, and is maintained in its contracted configuration.

3. The temporary stent of claim 1, wherein the strut is a rod-shaped body formed of a resin material including polytetrafluoroethylene, polyolefin, polyester, polyurethane or polysiloxane.

4. The temporary stent of any one of claims 1-3, wherein the filament is formed of a shape-memory material such that the first and second stent bodies each can be expanded to form cylindrical shapes as their normal state, the first, second and third proximal ends each can be tapered as their normal state, and the middle strut can be maintained in a contracted configuration as a normal state.

5. The temporary stent of claim 1 or 2, wherein the filament is formed of a shape-memory material such that the first and second stent bodies each can be expanded to form cylindrical shapes as their normal state, the first, second and third proximal ends each can be tapered as their normal state, and the middle strut and the strut each can be maintained in their contracted configurations as their normal state.

6. The temporary stent of claim 4 or 5, wherein said shape-memory material includes Ni-Ti based shape-memory alloy, Cu-Al-Ni based shape-memory alloy or Cu-Zn-Al based shape-memory alloy.

7. The temporary stent of any one of claims 1-3, wherein the filament is formed of a metal wire including stainless steel, titanium, nickel or tantalum.

8. The temporary stent of any one of claims 1-3, wherein the filament is formed of a plastic wire or fiber reinforced plastic wire, including polytetrafluoroethylene, polyolefin, polyester, polyurethane or polysiloxane.

9. The temporary stent of any one of claims 1-8, wherein the first stent body has a length of 5 cm to 10 cm, the first proximal end has a length of 2 cm to 3 cm, the middle strut has a length of 4 cm to 6 cm, the second proximal end has a length of 2 cm to 3 cm, the second stent body has a length of 5 cm to 10 cm, the third proximal end has a length of 4 cm to 6 cm, and the strut has a length of 60 cm to 100 cm.

10. The temporary stent of any one of claims 1-9, contained in a pipe-shaped catheter.

11. A temporary stent-graft, comprising:
the stent of any one of claims 1-10; and
a cylindrically shaped graft configured to cover the stent in the area ranging between the first stent body and the second stent body.

12. The temporary stent-graft of claim 11, wherein a distal end of the graft is coupled with a distal end of the stent by a connecting member in a manner that can be reversed.

13. The temporary stent-graft of claim 12, wherein the distal end of the stent is provided with a loop formed by turning around a plurality of filaments arranged at regular intervals, putting two filaments together end on end, and twisting a turn-around part of the filament

14. The temporary stent-graft of claim 12 or 13, wherein the connecting member is a suture.

15. The temporary stent-graft of any one of claims 11-14, contained in a pipe-shaped catheter.
